# EUROPEAN PATENT SPECIFICATION

(11) **EP 3 736 055 B1**
(45) Date of publication and mention of the grant of the patent: **01.03.2023**
(21) Application number: 20172211.3
(22) Date of filing: 29.04.2020
(51) Int. Cl.: B08B 3/04, B08B 9/00, B08B 9/08, A61L 2/18, A61B 90/70

(54) **METHOD TO CLEAN AN APPARATUS TO TREAT LOOSE OBJECTS AND CORRESPONDING APPARATUS**
VERFAHREN ZUR REINIGUNG EINER VORRICHTUNG ZUR BEHANDLUNG VON LOSEN GEGENSTÄNDEN UND ENTSPRECHENDE VORRICHTUNG
PROCÉDÉ DE NETTOYAGE D'UN APPAREIL POUR TRAITER DES OBJETS EN VRAC ET APPAREIL CORRESPONDANT

(30) Priority: 07.05.2019 IT 201900006611
(43) Date of publication of application: 11.11.2020
(73) Proprietor: Icos Pharma S.p.A., 33080 Zoppola (IT)
(72) Inventor: Beni, Stefano, 33170 Pordenone (IT)
(74) Representative: Petraz, Gilberto Luigi

(56) References cited:
- EP-A1- 2 060 275
- EP-A1- 3 406 268
- WO-A1-2015/150581
- WO-A1-2017/182545
- WO-A2-2018/225106
- US-B1- 6 770 150

## Description

### FIELD OF THE INVENTION

Embodiments described here concern a method to clean an apparatus to treat loose objects and a corresponding apparatus.

It is known that apparatuses to treat loose objects, for example to wash, and/or sterilize them, normally require cleaning, normally sterilizing, the apparatuses themselves, typically on a cyclical basis, for example before their first use or after a certain number of treatment cycles of the loose objects. For example, the loose objects in question can be caps for closing test tubes for laboratory analysis, caps for closing drug vials, or small instruments or objects used in operating rooms or laboratories, or other.

### BACKGROUND OF THE INVENTION

It is known that in the hospital, or pharmaceutical, or other sectors, for example the food sector, it is necessary to use sterile or suitably treated objects to prevent contamination or alterations of substances with which they come into contact, or to prevent introducing bacteria, germs, other corpuscles or potentially pathogenic or harmful agents into environments where their total absence is required.

The sterile objects as above normally include, in all the sectors listed above, loose objects, even small ones, such as, for example, caps for closing test tubes intended to contain substances to be subjected to laboratory analysis, or small instruments or objects used in operating rooms or laboratories.

As is known, in an apparatus to treat objects, the loading of the loose objects to be treated in the treatment machine takes place by means of a feed section which cooperates with a loading aperture located above the treatment machine.

After the treatment, the treated loose products are removed from a discharge mouth of the treatment machine and taken toward a discharge section. A use station, for example a filling machine, can be provided in the discharge section, or in contact therewith.

The discharge section and the filling machine have to be suitably cleaned, normally sterilized, since they are directly in contact with the objects treated by the treatment machine. Consequently, any contamination present in the discharge section and/or in the filling machine could invalidate the treatment of the objects.

The existing methods to clean, generally sterilize, treatment apparatuses do not always guarantee the complete separation between untreated, and therefore contaminated, environments, such as for example the feed section, and treated environments, normally sterile, such as for example the treatment machine and in particular the discharge zone in contact with the user machine.

Furthermore, the treatment apparatuses have generally complex known cleaning systems or assemblies, with consequent cleaning methods that are often laborious and expensive.

In addition, known cleaning methods often require, for their application, a high number of adaptations of the apparatus to treat loose objects with consequent substantial installation, maintenance and adaptation costs.

In document WO-A-2018/225106 a method and a sterilizing machine for loose products are described. In the method and machine of the document WO-A-2018/225106, a cleaning cycle by means of a cleaning fluid is performed when the apparatus is not being used to treat the loose objects, which, while said cleaning cycle is performed, are not present in the treatment apparatus.

Document WO-A-2017/182545 describes a reactor comprising a nozzle for cleaning fluid. Document EP-A-2060275 describes a steam sterilizing system.

Over the years, the need has arisen to obtain a method to clean an apparatus to treat loose objects and a corresponding apparatus that are increasingly optimized, safe, easy to use and controllable, in particular in the field of application of the pharmaceutical industry.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

There is therefore the need to perfect a method to clean an apparatus to treat loose objects and a corresponding apparatus that can overcome and improve the existing cleaning methods and apparatuses.

In particular, one purpose of the present invention is to provide a method and a corresponding apparatus to clean, in particular to sterilize, known apparatuses to treat loose objects which can be easily implemented on the latter.

Another purpose of the present invention is to define a method to clean known apparatuses to treat loose objects which guarantees the complete separation between contaminated environments and controlled or sterile environments.

Another purpose of the present invention is to define a method to clean and a corresponding apparatus to clean, in particular to sterilize, known apparatuses to treat loose objects which are efficient and optimized.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, a method, according to the invention, to clean with cleaning fluid an apparatus to treat loose objects provided with a machine to treat loose objects and a section to discharge the loose objects treated by the treatment machine toward a use station operatively and selectively in communication with the discharge section comprises performing a cleaning cycle when the treatment apparatus is not being used to treat the loose objects, which, while the cleaning cycle is performed, are not present in the treatment apparatus, the cleaning cycle comprising:
- delivering a cleaning fluid into the treatment apparatus using the same introduction means provided in the treatment machine, the cleaning fluid being made to flow along a cleaning circuit comprising the same passage circuit of the loose objects treated by the treatment apparatus which develops along the treatment machine and the discharge section up to an outlet selectively associated with the use station;
- discharging the cleaning fluid through a discharge circuit downstream of the discharge section and which is selectively connected to the outlet by means of a connection pipe only in a first operating condition, which is used to perform the cleaning cycle of the treatment apparatus, when a loading aperture of the treatment machine is sealed closed and the discharge section is in communication with the treatment machine and is not in communication with the use station, while in a second non-operating condition, which is used to treat the loose objects in the treatment apparatus, the discharge circuit is not connected to the outlet and the outlet is therefore in communication with the use station allowing the discharge of the loose objects into the use station by means of the outlet.

Advantageously, this method allows to use the treatment machine of the treatment apparatus itself to clean, preferably sterilize, the treatment apparatus simplifying cleaning operations, optimizing cleaning times and guaranteeing a complete separation between contaminated environments and controlled or sterile environments.

The present invention also concerns an apparatus to treat loose objects comprising:
- a treatment machine provided with a hermetically sealed treatment chamber in which to treat loose objects received through a loading aperture by means of a treatment fluid provided by introduction means provided in the treatment machine,
- a section to discharge treated loose objects connected downstream of the treatment machine by discharge means to discharge the treated loose objects at a use station in a controlled environment, the use station being operatively and selectively in communication with the discharge section by means of connection means,
- a passage circuit for the flow of loose objects which develops from the treatment chamber to an outlet of the connection means and comprising at least the treatment chamber, the discharge means, the discharge section and the connection means.

According to the invention, the apparatus comprises an assembly to clean the treatment apparatus to deliver cleaning fluid into the treatment apparatus by means of the same introduction means provided in the treatment machine, the cleaning assembly comprising:
- a cleaning circuit comprising the same the passage circuit, along which the cleaning fluid is able to flow;
- a circuit to discharge the cleaning fluid connected downstream of the cleaning circuit;
- a connection pipe connectable on one side to the discharge circuit and on the other to the outlet of the connection means, so that the connection pipe selectively has:

- a first operating condition, when the loading aperture is sealed closed and the discharge section is in communication with the treatment machine and is not in communication with the use station, which is used to perform the cleaning cycle of the treatment apparatus and in which the connection pipe connects the discharge circuit to the outlet of the connection means to discharge the cleaning fluid;
- a second non-operating condition, when the discharge section is in communication with the use station, which is used to treat the loose objects in the treatment apparatus and in which the connection pipe does not connect the discharge circuit to the outlet of the connection means, the outlet therefore being in communication with the use station, allowing the discharge of the loose objects into the use station by means of the outlet.

Advantageously, this solution provides to use the treatment machine to clean, preferably sterilize, the treatment apparatus without the need to adapt the latter or to use bulky additional apparatuses which are laborious to install.

Consequently, cleaning the apparatus to treat loose objects is significantly optimized.

### BRIEF DESCRIPTION OF THE DRAWINGS

These and other aspects, characteristics and advantages of the present invention will become apparent from the following description of some embodiments, given as a non-restrictive example with reference to the attached drawings wherein:
- figs. 1-2 are example schematic illustrations of variants of an apparatus to treat loose objects in accordance with the present invention;
- fig. 3 is an example diagram of another variant of figs. 1-2;
- fig. 4 is a variant of embodiments described here.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We will now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants insofar as these are within the scope of the appended claims.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

According to the invention, with reference to the attached drawings, a method to clean with cleaning fluid an apparatus 11 to treat loose objects provided with a machine 12 to treat the loose objects and with a section 13 to discharge the loose objects treated by the treatment machine 12 toward a use station 14 operatively and selectively in communication with the discharge section 13 comprises performing a cleaning cycle when the treatment apparatus 11 is not being used to treat the loose objects, which, while the cleaning cycle is performed, are not present in the treatment apparatus 11.

The cleaning cycle comprises:
- delivering a cleaning fluid into the treatment apparatus 11 using the same introduction means 24 provided in the treatment machine 12, the cleaning fluid being made to flow along a cleaning circuit 30 comprising the same passage circuit 20 of the loose objects treated by the treatment apparatus 11 which develops along the treatment machine 12 and the discharge section 13 up to an outlet 31 selectively associated with the use station 14;
- discharging the cleaning fluid through a discharge circuit 17 downstream of the discharge section 13 and which is selectively connected to the outlet 31 by means of a connection pipe 23 only in a first operating condition, which is used to perform the cleaning cycle of the treatment apparatus 11, when a loading aperture 16 of the treatment machine 12 is sealed closed and the discharge section 13 is in communication with the treatment machine 12 and is not in communication with the use station 14, while in a second non-operating condition, which is used to treat the loose objects in the treatment apparatus 11, the discharge circuit 17 is not connected to the outlet 31 and the outlet 31 is therefore in communication with the use station 14 allowing the discharge of the loose objects into the use station 14 by means of the outlet 31.

Advantageously, this method allows to adequately clean, preferably sterilize, the discharge section 13 before connecting it to the use station 14 which has a controlled environment, generally sterile, and before moving the treated loose objects inside it. In this way, the loose objects after the cleaning cycle will be in contact with a decontaminated environment, preferably sterile. This procedure is particularly sought after in the pharmaceutical field when the loose objects, once sterilized, have to always be in contact with a sterile environment.

According to one embodiment, during the cleaning cycle, the treatment machine 12 can be put in direct communication with the discharge section 13, for example by means of an aperture of an interception device 41 provided associated with discharge means 21 which connect the treatment machine 12 to the discharge section 13.

According to one embodiment, during the cleaning cycle, it is provided to clean the treatment machine 12, the discharge section 13 and at least the connection pipe 23.

In particular, during the cleaning cycle, the treatment machine 12, the discharge section 13 and at least the connection pipe 23 are put in fluidic communication with each other.

According to one embodiment, the discharge is performed through a main discharge pipe 25 of the discharge circuit 17 preventing the cleaning fluid from reaching the use station 14.

According to one embodiment, the method provides to check the status of the cleaning cycle and the correct configuration of the treatment apparatus 11 during the cleaning cycle by means of one or more temperature and pressure sensors 35 and a sensor 36 associated with the outlet 31 to detect the correct connection between the discharge circuit 17 and the outlet 31, the sensors 35, 36 being connected to a command and control unit 40.

By "correct configuration" of the treatment apparatus 11 we mean at least loading aperture 16 closed, treatment machine 12 and discharge section 13 in fluid communication and correct discharge of the cleaning fluid at the end of the cleaning cycle.

According to one embodiment, at the end of the cleaning cycle, it is provided to connect the discharge section 13 and the use station 14 in a controlled and sterile environment maintaining the loading aperture 16 of the treatment machine 12 sealed.

According to one embodiment, at the end of the cleaning cycle, it is provided to close the discharge means 21 which selectively put the treatment machine 12 in communication with the discharge section 13, by means of the interception device 41, before opening the loading aperture 16 of the treatment machine 12 so as to keep the discharge section 13 in a clean, preferably sterilized, condition.

In fact, since the elements downstream of the interception device 41 have been treated, preferably sterilized, in this cleaning cycle, before performing a treatment cycle with loose objects to be loaded into the treatment machine 12, the interception device 41 is preferably closed, so that, when the treatment machine 12 is gradually loaded with loose objects to be treated, all the elements that are downstream of the interception device 41 remain suitably treated, or sterile.

With reference to the attached drawings, an apparatus 11 to treat loose objects comprises the treatment machine 12 provided with a hermetically sealed treatment chamber 22 in which to treat loose objects received through the loading aperture 16.

According to one embodiment, the treatment machine 12 internally provides introduction means 24 for supplying treatment fluid into treatment chamber 22.

The apparatus 11 to treat loose objects also comprises the section 13 to discharge treated loose objects connected downstream of the treatment machine 12 by means of discharge means 21 to discharge the treated loose objects at the use station 14 in a controlled environment.

The use station 14 is operatively and selectively in communication with the discharge section 13 by means of connection means 15.

The apparatus 11 to treat loose objects also comprises a passage circuit 20 for the flow of loose objects which develops from the treatment chamber 22 to the outlet 31 of the connection means 15 and comprising at least the treatment chamber 22, the discharge means 21, the discharge section 13 and the connection means 15.

According to the invention, the treatment apparatus 11 comprises an assembly 10 to clean the treatment apparatus 11 to deliver cleaning fluid into the apparatus 11 by means of the same introduction means 24 provided in the treatment machine 12.

In particular, the cleaning assembly 10 comprises the cleaning circuit 30 comprising the same passage circuit 20, along which the cleaning fluid is able to flow.

In addition, the cleaning assembly 10 comprises the circuit 17 to discharge the cleaning fluid connected downstream of the cleaning circuit 30.

The cleaning assembly 10 comprises the connection pipe 23 connectable on one side to the discharge circuit 17 and on the other to the outlet 31 of the connection means 15, so that the connection pipe 23 selectively has:
- a first operating condition, when the loading aperture 16 is sealed closed and the discharge section 13 is in communication with the treatment machine 12 and is not in communication with the use station 14, which is used to perform the cleaning cycle of the treatment apparatus 11 and in which the connection pipe 23 connects the discharge circuit 17 to the outlet 31 of the connection means 15 to discharge the cleaning fluid;
- a second non-operating condition, when the discharge section 13 is in communication with the use station 14, which is used to perform the treatment of the loose objects in the treatment apparatus 11 and in which the connection pipe 23 does not connect the discharge circuit 17 to the outlet 31 of the connection means 15, the outlet 31 being therefore in communication with the use station 14 allowing the discharge of the loose objects into the use station 14 by means of the outlet 31.

With the terms "treatment", "treat" and "treated" we mean one or more washing and/or sterilization operations, with possible final drying. In the context of washing, it is possible, for example, to perform a pre-washing, washing with water or washing with chemicals, a combination of these washes, rinsing with hot or cold water. In the context of the treatment, a possible thermal disinfection operation is also contemplated. In the context of the treatment, it is also possible to surface treat the loose objects, for example by means of silicone coating; the silicone coating can be performed for example in the context of the washing operation. Another possible example is a treatment that includes drying, typically by means of controlled air, for example sterile. For example, a treatment that includes washing and drying is possible. Yet another possible example of treatment provides washing, sterilization and drying. In these examples, the surface treatment of the loose objects, for example silicone coating, can be performed at the same time as or after washing, before sterilization when provided. Yet another example of treatment provides sterilization and drying. Other combinations of the treatments as above can be contemplated in the embodiments described here.

By "cleaning" we mean one or more washing, rinsing and/or sterilization operations, with possible final drying of the treatment apparatus 11.

By "cleaning fluid" we mean washing, rinsing and/or sanitizing liquid, sanitizing vapors, drying flows (for example air or sterile gases).

In possible embodiments, the use station 14 can be a user machine, for example a filling machine, kept in controlled operating conditions, for example sterile, that is, it is sterile and works in a controlled and sterile atmosphere. The sterility of the user machine can normally be obtained by means of hot sterilization, or low temperature sterilization, for example Vaporized Hydrogen Peroxide (VHP) treatment.

The discharge section 13 is selectively connected to the treatment machine 12 by means of the interception device 41 which selectively inhibits the discharge means 21.

The interception device 41 is provided to selectively enable the transit of the loose objects, treated in the treatment machine 12, from the treatment machine 12 to the use station 14 and is suitable to selectively prevent a communication between the feed section and the discharge section 13 when the these are not in the same conditions of sterility.

The discharge means 21 can be comprised in the discharge section 13. The discharge section 13 can be associated, connected or integrated with the use station 14 as described above.

The use station 14 is operatively put in communication with the discharge section 13 by means of the connection means 15, for example a connection pipe.

According to one embodiment, in the first non-operating condition of the connection pipe 23 the treatment apparatus 11 is configured to perform a treatment cycle of the loose objects and a consequent discharge of the treated loose objects along the passage circuit 20 toward the use station 14.

According to another embodiment, in the second non-operating condition of the connection pipe 23 the treatment apparatus 11 is configured to perform a cleaning cycle of the treatment machine 12 and of the discharge section 13 by means of the cleaning assembly 10 and along the cleaning circuit 30.

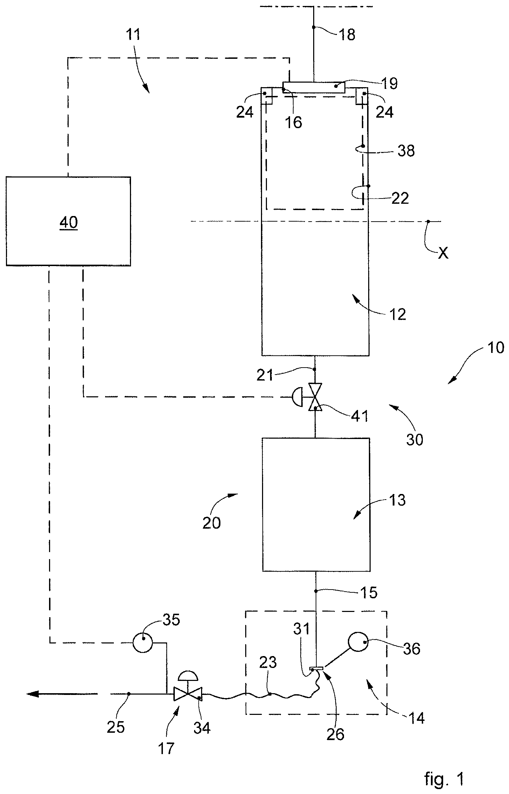

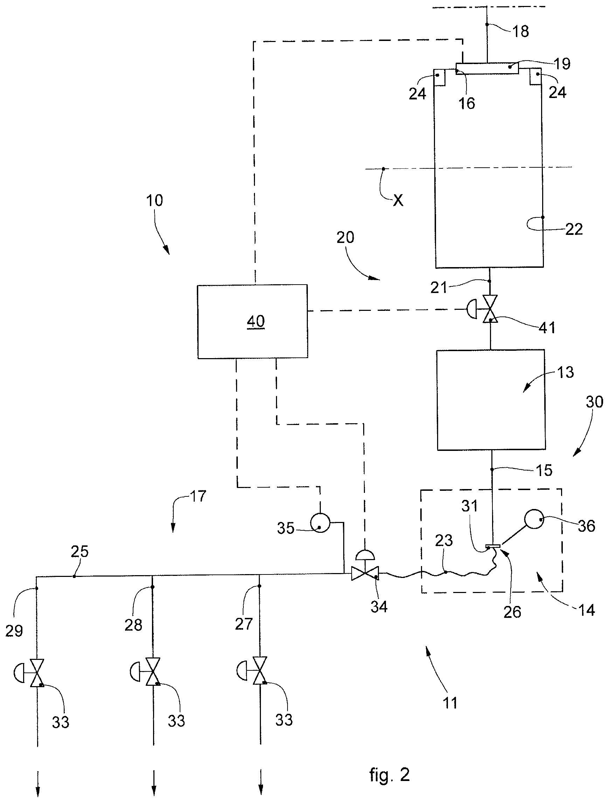

According to one embodiment, shown by way of example in figs. 1 and 2, the connection means 15 have a junction device 26 associated with the outlet 31 and configured to connect/disconnect the discharge circuit 17 to the connection means 15, or to the passage circuit 20, in order to define the cleaning circuit 30.

In particular, the junction device 26 connects the discharge circuit 17 to the connection means 15 in a hermetic and sealed manner.

According to one embodiment, in the first operating condition of the connection pipe 23 the junction device 26 is configured to selectively connect the use station 14 to the discharge section 13. In this operating condition, the treatment apparatus 11 is configured to treat the loose objects and selectively feed them, once treated, to the use station 14.

According to one embodiment, in the second non-operating condition of the connection pipe 23 the junction device 26 is configured to selectively disconnect the use station 14 from the discharge section 13 and to selectively hermetically connect the discharge section 13 to the discharge circuit 17. In this non-operating condition, loose objects are not moved, treated or discharged inside the cleaning assembly 10 and, therefore, in the cleaning circuit 30.

In the first operating condition as above, the means to introduce treatment fluid of the treatment machine 12 deliver treatment fluid for the treatment cycle of the loose objects present in the treatment machine 12.

In the second non-operating condition as above, the means to introduce cleaning fluid of the treatment machine 12 deliver cleaning fluid to perform a cleaning cycle along the cleaning circuit 30, in particular sterilization, of the treatment machine 12, of the discharge means 21, of the discharge section 14 and of the connection means 15 when there are no loose objects inside.

According to one embodiment, the treatment fluid and the cleaning fluid can be the same type of fluid.

In the second non-operating condition of the connection pipe 23 as above the interception device 41 is in an open position allowing the connection between the treatment machine 12 and the discharge section 13.

The discharge circuit 17 is configured to discharge the cleaning fluid at the end of the cleaning cycle of the treatment apparatus 11.

The cleaning fluid discharged can be in the form of liquids, gases, condensate or residual vapors after the cleaning cycle of the treatment apparatus 11.

According to one embodiment, the connection pipe 23 in its second non-operating configuration is configured to be connected to the junction device 26.

Advantageously, the connection pipe 23 can be a flexible tube which can be easily connected to the junction device 26 since it occupies a limited bulk, reducing to a minimum the adaptation interventions necessary for the treatment apparatus 11 when passing from the first operating configuration to the second non-operating configuration and vice versa.

According to one embodiment, the junction device 26 can be provided inside the use station 14 so that the connection between the discharge circuit 17 and the passage circuit 20 and, in particular, the connection between the passage circuit 20, once sterilized, and the use station 14 occurs in a controlled and sterile environment such as that of the use station 14.

According to one embodiment, the discharge circuit 17 is provided with at least one main discharge pipe 25 associated with the connection pipe by means of a shutter device 34, for example an interception valve.

The main discharge pipe 25 is configured to discharge outside the use station 14 the cleaning fluid used during the cleaning of the treatment apparatus 11 by means of the cleaning assembly 10.

According to one embodiment, the discharge circuit 17 comprises at least a first discharge pipe 27 connected to the main discharge pipe 25 and configured to discharge the discharge liquids generated during the cleaning cycle of the treatment apparatus 11.

According to one embodiment, the discharge circuit 17 comprises at least a second discharge pipe 28 connected to the main discharge pipe 25 and configured to discharge the discharge gases, for example the drying gases, sterile or other gases generated during the cleaning cycle of the treatment apparatus 11.

According to one embodiment, the discharge circuit 17 comprises at least a third discharge pipe 29 connected to the main discharge pipe 25 and configured to discharge the discharge vapors generated during the cleaning cycle of the treatment apparatus 11.

The third discharge pipe 29 can comprise a vapor condenser 37 for condensing the discharge vapors so as to eliminate them in a liquid state, and a vent device 32 for eliminating the gases that were not condensed in the vapor condenser 37.

With each of the first, second and third discharge pipes 27, 28, 29 there can be associated a discharge valve 33 to selectively allow the discharge through the discharge pipes 27, 28, 29.

According to one embodiment, the cleaning assembly 10 can comprise one or more sensors 35 for detecting the temperature and pressure of the cleaning fluid during the cleaning cycle and for checking the correct execution of the cleaning cycle.

The sensors 35 can be disposed, for example, at the level of the main discharge pipe 25 and/or of the junction device 26. However, other sensors 35 disposed at other points of the cleaning assembly 10 are not excluded.

In particular, the cleaning assembly 10 comprises the sensor 36 associated with the outlet 31 which also detects the correct connection between the discharge circuit 17 and the connection means 15 by means of the junction device 26 in the first operating configuration and between the use station 14 and the treatment apparatus 11 in the second non-operating configuration.

According to one embodiment, the sensor 36 can be associated with the junction device 26.

According to one embodiment, the cleaning assembly 10 comprises the command and control unit 40 configured to automatically manage the functioning of the cleaning assembly 10 during the cleaning cycle.

In particular, the activation of the discharge through the discharge circuit 17 and the management of the cleaning cycle by means of the treatment machine 12 are governed by the command and control unit 40.

According to one embodiment, the discharge circuit 17 is associated with the command and control unit 40 to monitor the progress of the cleaning cycle.

The sensors 35, 36 are associated with the command and control unit 40. On the basis of the detections of the sensors 35, the command and control unit 40 manages the cleaning cycle, adjusting the delivery of the cleaning fluid by the introduction means 24 and the discharge of the cleaning fluid through the discharge circuit 17 accordingly.

According to one embodiment, the loading aperture 16 of the treatment machine 12 can be selectively connected, by means of feed means 18, to a section (not shown) for feeding loose objects to be treated.

With reference to the attached drawings, the treatment machine 12 also comprises a closing device 19 associated with the loading aperture 16, which selectively closes the loading aperture 16 so as to hermetically seal the treatment machine 12 with respect to the feed section and, in particular, to the feed means 18.

In the first operating configuration of the connection pipe 23, the cleaning circuit 30 provides the closing device 19 in a closed position so as to seal the loading aperture 16 of the treatment machine 12.

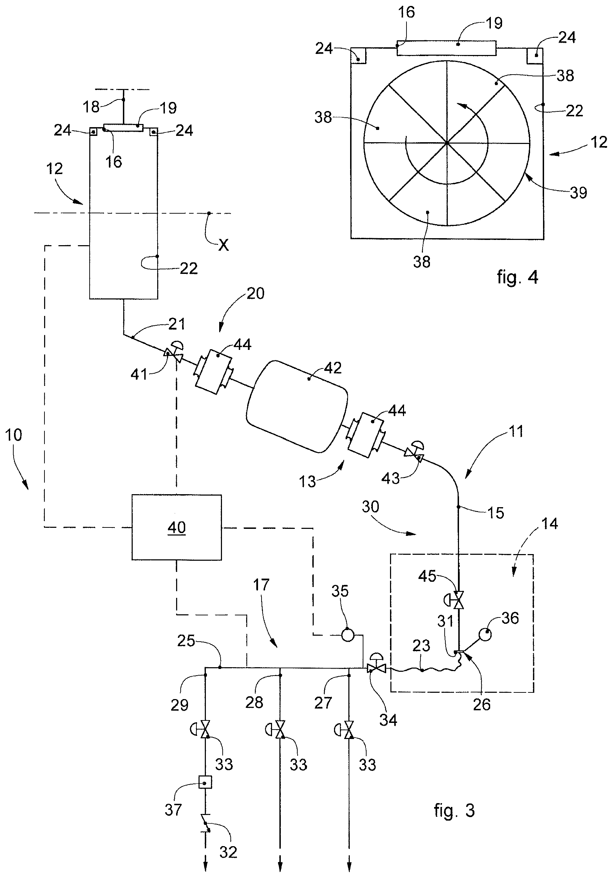

The treatment machine 12 can have a single treatment chamber 22, or a treatment chamber 22 divided into separate containing compartments or sectors 38. For example, with reference to fig. 4, the treatment chamber 22 can be defined by a drum 39 rotating around an axis of rotation X and the rotating drum 39 can have the containing compartments or sectors 38 separated and disposed angularly around the axis X.

Therefore, as shown by way of example in the attached drawings, the treatment chamber 22 can provide a single containing compartment or sector 38 or several containing compartments or sectors 38 (fig. 4) inside which the loose objects to be treated are disposed.

Treatment machines 12 of this type are described for example in the international application WO-A-2015150581 in the name of the Applicant.

However, other types of treatment machines 12 with one or more treatment chambers 22 and/or with one or more containing sectors 38 are not excluded.

According to one embodiment, the discharge section 13 can comprise at least one intermediate accumulation container 42 (fig. 2) in which the treated loose objects normally accumulate, before being transferred to the use station 14.

Possible embodiments of accumulation containers 42 are described by way of example in the international application WO 2018/225106 A2 in the name of the Applicant.

In particular, with reference to fig. 3, the interception device 41 is disposed upstream of the accumulation container 42. The interception device 41 can be put in cooperation with the discharge means 21. Downstream of the accumulation container 42 another interception device 43 can be provided (fig. 3) to selectively intercept or release the treated loose objects toward the use station 14.

According to one embodiment, the accumulation container 42 can be rotatable around its longitudinal axis to allow the movement of the loose objects and can be connected to the discharge means 21 and to the connection means 15 by means of one or more rotary joints 44 (fig 3) which allow the relative rotation of the accumulation container 42 with respect to the discharge means 21 and to the connection means 15.

During the cleaning cycle, the accumulation container 42 is advantageously cleaned, preferably sterilized, by the treatment machine 12 since the latter is in direct communication with the outlet 31 of the connection means 15 along the passage circuit 20.

According to one embodiment, the treatment apparatus 11 comprises a selector device 45 put in cooperation with the connection means 15 upstream of the outlet 31 and, therefore, of the use station 14.

The selector device 45 is configured to distribute the loose objects to the use station 14 in a controlled and homogeneous manner.

The selector device 45 can be a motorized valve provided, for example, with one or more blades able to slow down the flow of loose objects transferred, gradually distributing and feeding the same loose objects to the use station 14.

During the cleaning cycle, the selector device 45 is advantageously cleaned, preferably sterilized, by the treatment machine 12 since the latter is in direct communication with the outlet 31 of the connection means 15 along the passage circuit 20.

The command and control unit 40 can comprise a central processing unit, or CPU, an electronic memory, an electronic database and auxiliary circuits (or I/O) (not shown).

For example, the CPU can be any form of computer processor that can be used in the information technology sector to manage cleaning and treatment processes. The memory can be connected to the CPU and can be one or more of those commercially available, such as a random access memory (RAM), a read only memory (ROM), a floppy disk, a hard disk, a mass memory, or any other form of digital archiving, local or remote. The software instructions and data can for example be coded and stored in the memory to comand the CPU. The auxiliary circuits can also be connected to the CPU to help the processor in a conventional manner. The auxiliary circuits can include for example at least one of: cache circuits, power supply circuits, clock circuits, input/output circuitry, subsystems, and suchlike. A program (or computer instructions) readable by the command and control unit 40 can determine which tasks are achievable in accordance with the method according to the present description. In some embodiments, the program is a software readable by the command and control unit. The command and control unit includes a code to generate and store information and data introduced or generated during the method in accordance with the present description.

Some embodiments can provide the execution of various steps, passages and operations, as described above. These steps, passages and operations can be carried out with instructions executed by a machine that cause the execution of certain steps by a general-purpose or special-purpose processor. Alternatively, these steps, passages and operations can be performed by specific hardware components which contain hardware logic to carry out the steps, or by any combination of programmed computer components and customized hardware components.

Embodiments of the method in accordance with the present description can be included in a computer program which can be stored in a computer-readable medium which contains the instructions which, once carried out by the treatment apparatus 11, determine the execution of the method being discussed.

In particular, elements according to the present invention can be supplied as machine-readable media for storing the instructions executable by the machine. The machine-readable media can include, but are not limited to, floppy disks, optical disks, CD-ROMs, and magneto-optical disks, ROMs, RAMs, EPROMs, EEPROMs, optical or magnetic boards, propagation media or other types of machine-readable media suitable to store electronic information. For example, the present invention can be downloaded as a computer program that can be transferred from a remote computer (for example a server) to a computer that submits a request (for example client), by means of data signals made with wave carriers or other propagation means, via a communication link (for example a modem or a network connection).

It is clear that modifications and/or additions of parts or steps may be made to the method to clean an apparatus 11 to treat loose objects and the corresponding treatment apparatus 11 as described heretofore, without departing from the field and scope of the annexed claims.

It is also clear that, although the present invention has been described with reference to some specific examples, a person of skill in the art shall certainly be able to achieve many other equivalent forms of a method to clean an apparatus 11 to treat loose objects and a corresponding treatment apparatus 11, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Method to clean with cleaning fluid an apparatus (11) to treat loose objects provided with a machine (12), to treat said loose objects and comprising introduction means (24), a section (13) to discharge the loose objects treated by said treatment machine (12) toward a use station (14) operatively and selectively in communication with said discharge section (13),
wherein said method comprises performing a cleaning cycle when said treatment apparatus (11) is not being used to treat said loose objects, which, while said cleaning cycle is performed, are not present in said treatment apparatus (11), said cleaning cycle comprising:
- delivering a cleaning fluid into said treatment apparatus (11) using the same introduction means (24) provided in said treatment machine (12), said cleaning fluid being made to flow along a cleaning circuit (30) comprising the same passage circuit (20) of the loose objects treated by said treatment apparatus (11) which develops along said treatment machine (12) and said discharge section (13) up to an outlet (31) selectively associated with said use station (14);
- discharging said cleaning fluid through a discharge circuit (17) downstream of said discharge section (13) and which is selectively connected to said outlet (31) by means of a connection pipe (23) only in a first operating condition, which is used to perform the cleaning cycle of said treatment apparatus (11), when a loading aperture (16) of said treatment machine (12) is sealed closed and said discharge section (13) is in communication with said treatment machine (12) and is not in communication with said use station (14), while in a second non-operating condition, which is used to treat the loose objects in said treatment apparatus (11), said discharge circuit (17) is not connected to said outlet (31) by means of said connection pipe (23) and said outlet (31) is therefore in communication with said use station (14) allowing the discharge of said loose objects into said use station (14) by means of said outlet (31).

2. Method as in claim 1, **characterized in that** said discharge is performed through a main discharge pipe (25) of the discharge circuit (17) preventing the cleaning fluid from reaching the use station (14).

3. Method as in claim 1 or 2, **characterized in that** it provides to check the state of the cleaning cycle and the correct configuration of the treatment apparatus (11) during said cleaning cycle by means of one or more temperature and pressure sensors (35) and a sensor (36) associated with the outlet (31) to detect the correct connection between said discharge circuit (17) and said outlet (31), said sensors (35, 36) being connected to a command and control unit (40).

4. Method as in any claim hereinbefore, **characterized in that,** at the end of the cleaning cycle, it is provided to connect the discharge section (13) and the use station (14) in a controlled environment maintaining the loading aperture (16) of the treatment machine (12) sealed.

5. Method as in any claim hereinbefore, **characterized in that,** at the end of the cleaning cycle, it is provided to close discharge means (21) which selectively put the treatment machine (12) in communication with said discharge section (13) before opening the loading aperture (16) of the treatment machine (12).

6. Apparatus (11) to treat loose objects comprising:
a treatment machine (12) provided with a hermetically sealed treatment chamber (22) in which to treat loose objects received through a loading aperture (16) by means of a treatment fluid provided by introduction means (24) provided in said treatment machine (12),
a section (13) to discharge treated loose objects connected downstream of said treatment machine (12) by discharge means (21) to discharge the treated loose objects at a use station (14) in a controlled environment, said use station (14) being operatively and selectively in communication with said discharge section (13) by means of connection means (15),
a passage circuit (20) for the flow of loose objects which develops from said treatment chamber (22) to an outlet (31) of said connection means (15) and comprising at least said treatment chamber (22), said discharge means (21), said discharge section (13) and said connection means (15),
wherein said apparatus comprises an assembly (10) to clean said treatment apparatus (11) to deliver cleaning fluid into said treatment apparatus (11) by means of the same introduction means (24) provided in said treatment machine (12), said cleaning assembly (10) comprising:
a cleaning circuit (30) comprising the same said passage circuit (20), along which said cleaning fluid is able to flow;
a circuit (17) to discharge said cleaning fluid connected downstream of said cleaning circuit (30);
a connection pipe (23) connectable on one side to said discharge circuit (17) and on the other to said outlet (31) of said connection means (15), so that said connection pipe (23) selectively has:
a first operating condition, when said loading aperture (16) is sealed closed and
said discharge section (13) is in communication with said treatment machine (12) and is not in communication with said use station (14), which is used to perform the cleaning cycle of said treatment apparatus (11) and in which said connection pipe (23) connects said discharge circuit (17) to said outlet (31) of said connection means (15) to discharge said cleaning fluid;
a second non-operating condition, when said discharge section (13) is in communication with said use station (14), which is used to treat the loose objects in said treatment apparatus (11) and in which the connection pipe (23) does not connect said discharge circuit (17) to said outlet (31) of said connection means (15), said outlet (31) therefore being in communication with said use station (14), allowing the discharge of said loose objects into said use station (14) by means of said outlet (31).

7. Apparatus as in claim 6, **characterized in that** said connection pipe (23) is a flexible tube.

8. Apparatus as in claim 6 or 7, **characterized in that** said discharge circuit (17) is provided with at least a main discharge pipe (25) associated with said connection pipe (23) by a shutter device (34) and configured to discharge the cleaning fluid used during cleaning outside the use station (14).

9. Apparatus as in any claim from 6 to 8, **characterized in that** said cleaning assembly (10) comprises a command and control unit (40) configured to automatically manage the functioning of the cleaning assembly (10) during a cleaning cycle, said cleaning assembly (10) having temperature and pressure sensors (35) associated with said command and control unit (40).

10. Apparatus as in any claim from 6 to 9, **characterized in that** said cleaning assembly (10) comprises a sensor (36) associated with the outlet (31) to detect the correct connection between said discharge circuit (17) and said connection means (15) by means of a junction device (26) in the first operating configuration and between said use station (14) and said treatment apparatus (11) in the second non-operating configuration.

11. Computer program storable in a medium readable by a computer containing the instructions which, once performed by an apparatus as in any claim from 6 to 10, determine the execution of the method as in any claim from 1 to 5.

## Patentansprüche

1. Verfahren zum Reinigen, mit Reinigungsfluid, einer Vorrichtung (11) zum Behandeln loser Objekte, welche bereitgestellt ist mit einer Maschine (12), um die losen Objekte zu behandeln und aufweisend Zuführmittel (24), einem Abschnitt (13), um die losen Objekte, die von der Behandlungsmaschine (12) behandelt wurden, zu einer Verwendungsstation (14) hin abzuführen, die betriebsmäßig und selektiv in Kommunikation mit dem Abführabschnitt (13) ist,
wobei das Verfahren aufweist Durchführen eines Reinigungszyklus, wenn die Behandlungsvorrichtung (11) nicht verwendet wird, um lose Objekte zu behandeln, welche, während der Reinigungszyklus durchgeführt wird, nicht in der Behandlungsvorrichtung (11) vorliegen, wobei der Reinigungszyklus aufweist:
- Zuführen eines Reinigungsfluid in die Behandlungsvorrichtung (11) unter Verwendung der selben Zuführmittel (24), die in der Behandlungsmaschine (12) bereitgestellt sind, wobei das Reinigungsfluid gemacht ist, um entlang eines Reinigungskreislaufs (30) zu strömen, der den selben Durchgangskreislauf (20) der losen Objekte aufweist, die von der Behandlungsvorrichtung (11) behandelt werden, welcher sich entlang der Behandlungsmaschine (12) und dem Abführabschnitt (13) bis zu einem Auslass (31) erstreckt, der selektiv mit der Verwendungsstation (14) verknüpft ist,
- Abführen des Reinigungsfluid durch einen Abführkreislauf (17), der dem Abführabschnitt (13) nachgeordnet ist und der selektiv mit dem Auslass (31) verbunden ist mittels eines Verbindungsrohrs (23) nur in einem ersten Arbeitszustand, welcher verwendet wird, um den Reinigungszyklus der Behandlungsvorrichtung (11) durchzuführen, wenn eine Ladeöffnung (16) der Behandlungsmaschine (12) dicht verschlossen ist und der Abführabschnitt (13) in Kommunikation mit der Behandlungsmaschine (12) ist und nicht in Kommunikation mit der Verwendungsstation (14) ist, wobei in einem zweiten Nicht-Arbeitszustand, welcher verwendet wird, um die losen Objekte in der Behandlungsvorrichtung (11) zu behandeln, der Abführkreislauf (17) nicht mit dem Auslass (31) verbunden ist mittels des Verbindungsrohrs (23) und der Auslass (31) daher in Kommunikation mit der Verwendungsstation (14) ist, wodurch die Abfuhr der losen Objekte in die Verwendungsstation (14) ermöglicht ist mittels des Auslasses (31).

2. Verfahren gemäß Anspruch 1, **dadurch gekennzeichnet, dass** die Abfuhr durchgeführt wird durch ein Hauptabführrohr (25) des Abführkreislaufs (17), wodurch das Reinigungsfluid gehindert wird, zur Verwendungsstation (14) zu gelangen.

3. Verfahren gemäß Anspruch 1 oder 2, **dadurch gekennzeichnet, dass** es bereitstellt, den Zustand des Reinigungszyklus und die korrekte Konfiguration der Behandlungsvorrichtung (11) während des Reinigungszyklus zu prüfen mittels eines oder mehrerer Temperatur- und Drucksensoren (35) und einem Sensor (36), der mit dem Auslass (31) verknüpft ist, um die korrekte Verbindung zwischen dem Auslasskreislauf (17) und dem Auslass (31) zu erfassen, wobei die Sensoren (35, 36) mit einer Befehls- und Steuereinheit (40) verbunden sind.

4. Verfahren gemäß irgendeinem vorigen Anspruch, **dadurch gekennzeichnet, dass** es am Ende des Reinigungszyklus bereitgestellt ist, den Abführabschnitt (13) und die Verwendungsstation (14) zu verbinden in einem gesteuerten Umfeld, die Ladeöffnung (16) der Behandlungsmaschine (12) abgedichtet aufrechterhaltend.

5. Verfahren gemäß irgendeinem vorigen Anspruch, **dadurch gekennzeichnet, dass** es am Ende des Reinigungszyklus bereitgestellt ist, Abführmittel (21) zu schließen, die die Behandlungsmaschine (12) selektiv in Kommunikation mit dem Abführabschnitt (13) bringen, vor dem Öffnen der Ladeöffnung (16) der Behandlungsmaschine (12).

6. Vorrichtung (11) zum Behandeln loser Objekte, aufweisend:
eine Behandlungsvorrichtung (12), die mit einer hermetisch abgedichteten Behandlungskammer (22) bereitgestellt ist, in welcher lose Objekte zu behandeln sind, die durch eine Ladeöffnung (16) empfangen werden, mittels eines Behandlungsfluid, das durch Zuführmittel (24) bereitgestellt wird, die in der Behandlungsmaschine (12) bereitgestellt sind,
einen Abschnitt (13) zum Abführen behandelter loser Objekte, der der Behandlungsmaschine (12) nachgeordnet ist, mittels Abführmitteln (21) zum Abführen der behandelten losen Objekte an einer Verwendungsstation (14) in einem gesteuerten Umfeld, wobei die Verwendungsstation (14) betriebsmäßig und selektiv in Kommunikation mit dem Abführabschnitt (13) ist mittels Verbindungsmitteln (15),
einen Durchgangskreislauf (20) für den Strom loser Objekte, welcher sich von der Behandlungskammer (22) aus zu einem Auslass (31) der Verbindungsmittel (15) erstreckt und aufweist wenigstens die Behandlungskammer (22), die Abführmittel (21), den Abführabschnitt (13) und die Verbindungsmittel (15),
wobei die Vorrichtung eine Einrichtung (10) zum Reinigen der Behandlungsvorrichtung (11) aufweist, um Reinigungsfluid in die Behandlungsvorrichtung (11) zuzuführen mittels der selben Zuführmittel (24), die in der Reinigungsmaschine (12) bereitgestellt sind, wobei die Reinigungseinrichtung (10) aufweist:
einen Reinigungskreislauf (30), der den selben Durchgangskreislauf (20) aufweist, entlang dem das Reinigungsfluid strömen kann,
einen Kreislauf (17) zum Abführen des Reinigungsfluid, der dem Reinigungskreislauf (30) nachgeordnet angeschlossen ist,
ein Verbindungsrohr (23), das an einer Seite mit dem Abführkreislauf (17) und an der anderen mit dem Auslass (31) der Verbindungsmittel (15) verbindbar ist, sodass das Verbindungsohr (23) selektiv hat:
einen ersten Arbeitszustand, wenn die Ladeöffnung (16) dicht verschlossen ist und der Abführabschnitt (13) in Kommunikation mit der Behandlungsmaschine (12) ist und nicht in Kommunikation mit der Verwendungsstation (14) ist, welcher verwendet wird, um den Reinigungszyklus der Behandlungsvorrichtung (11) durchzuführen und in welchem das Verbindungsrohr (23) den Abführkreislauf (17) mit dem Auslass (31) der Verbindungsmittel (15) verbindet, um das Reinigungsfluid abzuführen,
einen zweiten Nicht-Arbeitszustand, wenn der Abführabschnitt (13) in Kommunikation mit der Verwendungsstation (14) ist, welcher verwendet wird, um die losen Objekte in der Behandlungsstation (11) zu behandeln, und in welchem das Verbindungsrohr (23) den Abführkreislauf (17) nicht mit dem Auslass (31) der Verbindungsmittel (15) verbindet, wobei der Auslass (31) daher in Kommunikation mit der Verwendungsstation (14) ist, wodurch die Abfuhr der losen Objekte in die Verwendungsstation (14) ermöglicht ist mittels des Auslasses (31).

7. Vorrichtung gemäß Anspruch 6, **dadurch gekennzeichnet, dass** das Verbindungsrohr (23) ein flexibles Rohr ist.

8. Vorrichtung gemäß Anspruch 6 oder 7, **dadurch gekennzeichnet, dass** der Abführkreislauf (17) mit wenigstens einem Hauptabführrohr (25) bereitgestellt ist, das mit dem Verbindungsrohr (23) verknüpft ist durch eine Verschlussvorrichtung (34) und konfiguriert ist, um das während des Reinigens verwendete Reinigungsfluid nach außerhalb der Verwendungsstation (14) abzuführen.

9. Vorrichtung gemäß irgendeinem Anspruch von 6 bis 8, **dadurch gekennzeichnet, dass** die Reinigungseinrichtung (10) eine Befehls- und Steuereinheit (40) aufweist, die konfiguriert ist, um das Funktionieren der Reinigungseinrichtung (10) während eines Reinigungszyklus automatisch zu verwalten, wobei die Reinigungseinrichtung (10) Temperatur- und Drucksensoren (35) hat, die mit der Befehls- und Steuereinheit (40) verknüpft sind.

10. Vorrichtung gemäß irgendeinem Anspruch von 6 bis 9, **dadurch gekennzeichnet, dass** die Reinigungseinrichtung (10) einen Sensor (36) aufweist, der mit dem Auslass (31) verknüpft ist zum Erfassen der korrekten Verbindung zwischen dem Abführkreislauf (17) und den Verbindungsmitteln (15) mittels einer Verbindungsvorrichtung (26) in der ersten Arbeitskonfiguration und zwischen der Verwendungsstation (14) und der Behandlungsvorrichtung (11) in der zweiten Nicht-Arbeitskonfiguration.

11. Computerprogramm, das auf einem Medium speicherbar ist, das von einem Computer lesbar ist, aufweisend die Instruktionen, welche, wenn durch eine Vorrichtung gemäß irgendeinem Anspruch von 6 bis 10 durchgeführt, die Durchführung des Verfahrens gemäß irgendeinem Anspruch von 1 bis 5 bestimmen.

## Revendications

1. Procédé de nettoyage, à l'aide d'un fluide de nettoyage, d'un appareil (11) pour traiter des objets en vrac pourvu d'une machine (12) pour traiter lesdits objets en vrac, et comprenant des moyens d'introduction (24), une section (13) pour décharger les objets en vrac traités par ladite machine de traitement (12) vers un poste d'utilisation (14) de manière opérationnelle et sélective en communication avec ladite section de décharge (13),
dans lequel ledit procédé comprend l'exécution d'un cycle de nettoyage lorsque ledit appareil de traitement (11) n'est pas utilisé pour traiter lesdits objets en vrac qui, tandis que ledit cycle de nettoyage est effectué, ne sont pas présents dans ledit appareil de traitement (11), ledit cycle de nettoyage comprenant les étapes consistant à :
- délivrer un fluide de nettoyage dans ledit appareil de traitement (11) en utilisant les mêmes moyens d'introduction (24) prévus dans ladite machine de traitement (12), ledit fluide de nettoyage étant amené à s'écouler le long d'un circuit de nettoyage (30) comprenant le même circuit de passage (20) des objets en vrac traités par ledit appareil de traitement (11) qui se développe le long de ladite machine de traitement (12) et de ladite section de décharge (13) jusqu'à une sortie (31) associée de manière sélective vers ledit poste d'utilisation (14) ;
- décharger ledit fluide de nettoyage à travers un circuit de décharge (17) en aval de ladite section de décharge (13) et qui est connecté de manière sélective à ladite sortie (31) au moyen d'un tuyau de connexion (23) uniquement dans une première condition de fonctionnement, qui est utilisée pour effectuer le cycle de nettoyage dudit appareil de traitement (11), lorsqu'une ouverture de chargement (16) de ladite machine de traitement (12) est fermée de manière étanche et lorsque ladite section de décharge (13) est en communication avec ladite machine de traitement (12) et n'est pas en communication avec ledit poste d'utilisation (14), tandis que dans une seconde condition de non fonctionnement, qui est utilisée pour traiter les objets en vrac dans ledit appareil de traitement (11), ledit circuit de décharge (17) n'est pas connecté à ladite sortie (31) au moyen dudit tuyau de connexion (23) et ladite sortie (31) est par conséquent en communication avec ledit poste d'utilisation (14) en permettant la décharge desdits objets en vrac dans ledit poste d'utilisation (14) au moyen de ladite sortie (31).

2. Procédé selon la revendication 1, **caractérisé en ce que** ladite décharge est effectuée à travers un tuyau de décharge principal (25) du circuit de décharge (17), en empêchant le fluide de nettoyage d'atteindre le poste d'utilisation (14).

3. Procédé selon la revendication 1 ou 2, **caractérisé en ce qu'**il prévoit de vérifier l'état du cycle de nettoyage et la configuration correcte de l'appareil de traitement (11) pendant ledit cycle de nettoyage au moyen d'un ou plusieurs capteurs de température et de pression (35) et d'un capteur (36) associé à la sortie (31) pour détecter la connexion correcte entre ledit circuit de décharge (17) et ladite sortie (31), lesdits capteurs (35, 36) étant connectés à une unité d'instruction et de commande (40).

4. Procédé selon l'une quelconque des revendications précédentes, **caractérisé en ce qu'**à la fin du cycle de nettoyage, il est prévu de connecter la section de décharge (13) et le poste d'utilisation (14) dans un environnement commandé en maintenant l'ouverture de chargement (16) de la machine de traitement (12) fermée de manière étanche.

5. Procédé selon l'une quelconque des revendications précédentes, **caractérisé en ce qu'**à la fin du cycle de nettoyage, il est prévu de fermer des moyens de décharge (21) qui mettent de manière sélective la machine de traitement (12) en communication avec ladite section de décharge (13) avant d'ouvrir l'ouverture de chargement (16) de la machine de traitement (12).

6. Appareil (11) pour traiter des objets en vrac, comprenant :
une machine de traitement (12) pourvue d'une chambre de traitement fermée de manière étanche (22) pour traiter des objets en vrac reçus à travers une ouverture de chargement (16) au moyen d'un fluide de traitement fourni par des moyens d'introduction (24) prévus dans ladite machine de traitement (12),
une section (13) pour décharger des objets en vrac traités connectée en aval de ladite machine de traitement (12) par des moyens de décharge (21) pour décharger les objets en vrac traités au niveau d'un poste d'utilisation (14) dans un environnement commandé, ledit poste d'utilisation (14) étant en communication de manière opérationnelle et sélective avec ladite section de décharge (13) au moyen de moyens de connexion (15),
un circuit de passage (20) pour la circulation d'objets en vrac qui se développe depuis ladite chambre de traitement (22) vers une sortie (31) desdits moyens de connexion (15) et comprenant au moins ladite chambre de traitement (22), lesdits moyen de décharge (21), ladite section de décharge (13) et lesdits moyens de connexion (15),
dans lequel ledit appareil comprend un ensemble (10) pour nettoyer ledit appareil de traitement (11) afin de délivrer un fluide de nettoyage dans ledit appareil de traitement (11) au moyen des mêmes moyens d'introduction (24) prévus dans ladite machine de traitement (12), ledit ensemble de nettoyage (10) comprenant :
un circuit de nettoyage (30) comprenant le même circuit de passage (20), le long duquel ledit fluide de nettoyage est capable de s'écouler ;
un circuit (17) pour décharger ledit fluide de nettoyage connecté en aval dudit circuit de nettoyage (30) ;
un tuyau de connexion (23) pouvant être connecté d'un côté audit circuit de décharge (17) et de l'autre à ladite sortie (31) desdits moyens de connexion (15), de sorte que ledit tuyau de connexion (23) présente sélectivement :
une première condition de fonctionnement, lorsque ladite ouverture de chargement (16) est fermée de manière étanche et ladite section de décharge (13) est en communication avec ladite machine de traitement (12) et n'est pas en communication avec ledit poste d'utilisation (14), qui est utilisée pour effectuer le cycle de nettoyage dudit appareil de traitement (11) et dans laquelle ledit tuyau de connexion (23) connecte ledit circuit de décharge (17) à ladite sortie (31) desdits moyens de connexion (15) pour décharger ledit fluide de nettoyage ;
une seconde condition de non fonctionnement, lorsque ladite section de décharge (13) est en communication avec ledit poste d'utilisation (14), qui est utilisée pour traiter les objets en vrac dans ledit appareil de traitement (11) et dans laquelle le tuyau de connexion (23) ne connecte pas ledit circuit de décharge (17) à ladite sortie (31) desdits moyens de connexion (15), ladite sortie (31) étant donc en communication avec ledit poste d'utilisation (14), en permettant la décharge desdits objets en vrac dans ledit poste d'utilisation (14) au moyen de ladite sortie (31).

7. Appareil selon la revendication 6, **caractérisé en ce que** ledit tuyau de connexion (23) est un flexible.

8. Appareil selon la revendication 6 ou 7, **caractérisé en ce que** ledit circuit de décharge (17) est pourvu d'au moins un tuyau de décharge principal (25) associé audit tuyau de connexion (23) par un dispositif d'obturation (34) et configuré pour décharger le fluide de nettoyage utilisé pendant un nettoyage à l'extérieur du poste d'utilisation (14).

9. Appareil selon l'une quelconque des revendications 6 à 8, **caractérisé en ce que** ledit ensemble de nettoyage (10) comprend une unité de commande et d'instruction (40) configurée pour gérer automatiquement le fonctionnement de l'ensemble de nettoyage (10) pendant un cycle de nettoyage, ledit ensemble de nettoyage (10) présentant des capteurs de température et de pression (35) associés à ladite unité de commande et d'instruction (40).

10. Appareil selon l'une quelconque des revendications 6 à 9, **caractérisé en ce que** ledit ensemble de nettoyage (10) comprend un capteur (36) associé à la sortie (31) pour détecter la connexion correcte entre ledit circuit de décharge (17) et lesdits moyens de connexion (15) au moyen d'un dispositif de jonction (26) dans la première configuration de fonctionnement et entre ledit poste d'utilisation (14) et ledit appareil de traitement (11) dans la seconde configuration de non fonctionnement.

11. Programme d'ordinateur pouvant être stocké dans un support lisible par un ordinateur contenant les instructions qui, une fois exécutées par un appareil selon l'une quelconque des revendications 6 à 10, déterminent l'exécution du procédé selon l'une quelconque des revendications 1 à 5.
